# EUROPEAN PATENT SPECIFICATION

(11) **EP 3 901 174 B1**
(45) Date of publication and mention of the grant of the patent: **21.10.2026**
(21) Application number: 19900009.2
(22) Date of filing: 17.12.2019
(51) Int. Cl.: C07K 16/28, G01N 33/569, C07K 16/42

(54) **MONOCLONAL ANTIBODY AGAINST CD19 AND APPLICATION THEREOF**
MONOKLONALER ANTIKÖRPER GEGEN CD19 UND ANWENDUNG DAVON
ANTICORPS MONOCLONAL CIBLANT CD19 ET SON APPLICATION

(30) Priority: 20.12.2018 CN 201811565418
(43) Date of publication of application: 27.10.2021
(73) Proprietor: Bioswan Laboratories, Inc., Shanghai 200120 (CN)
(72) Inventor: DA, Liang, Shanghai 200120 (CN)
(74) Representative: Ipsilon
(86) International application number: PCT/CN2019/126087
(87) International publication number: WO 2020/125653

(56) References cited:
- WO-A1-2014/190273
- WO-A1-2014/190273
- CN-A- 108 490 174
- CN-A- 108 490 174
- CN-A- 108 508 200
- CN-A- 108 508 200
- YOSHIAKI SOHMA ET AL: "Accumulation of plasma cells in atherosclerotic lesions of Wa- tanabe heritable hyperlipidemic rabbits", MEDICAL SCIENCES, 31 May 1995 (1995-05-31), pages 4937 - 4941, XP055721501, Retrieved from the Internet <URL:https://www.ncbi.nlm.nih.gov/pmc/articles/PMC41822/pdf/pnas01487-0226.pdf> [retrieved on 20200811]
- BIPULENDU JENA ET AL: "Chimeric Antigen Receptor (CAR)-Specific Monoclonal Antibody to Detect CD19-Specific T Cells in Clinical Trials", PLOS ONE, vol. 8, no. 3, 1 March 2013 (2013-03-01), pages e57838, XP055122892, DOI: 10.1371/journal.pone.0057838
- CARMEN S M YONG ET AL: "CAR T-cell therapy of solid tumors", IMMUNOLOGY AND CELL BIOLOGY, vol. 95, no. 4, 22 December 2016 (2016-12-22), AU, pages 356 - 363, XP055380406, ISSN: 0818-9641, DOI: 10.1038/icb.2016.128
- SOHMA, Y.: "Accumulation of plasma cells in atherosclerotic lesions of Wa- tanabe heritable hyperlipidemic rabbits", PROC. NATL. ACAD. SCI. USA, 31 May 1995 (1995-05-31), XP055721501
- DATABASE NCBI GENBANK 27 March 2002 (2002-03-27), SOHMA, Y: "Immunoglobulin kappa chain [Oryctolagus cuniculus", Database accession no. BAA07455.1
- DE OLIVEIRA, S. N.: "A CD 19/Fc fusion protein for detection of anti- CD 19 chimeric antigen receptors", JOURNAL OF TRANSLATIONAL MEDICINE, vol. 11, 29 January 2013 (2013-01-29), XP021146986, DOI: 10.1186/1479-5876-11-23

## Description

### Technical field

The invention belongs to the field of biotechnology and medicine, in particular to a monoclonal antibody which anti-CD19 antibody and an application thereof.

### Background

With the development of tumor immunotherapy, chimeric antigen receptor (CAR)-T cell immunotherapy, which combines the advantages of antibodies and immune cells, has attracted great attention. CAR is mainly composed of extracellular antigen binding region and intracellular signal transduction region through hinge region and transmembrane region. The extracellular antigen binding region has the function of specifically recognizing and binding the target cell surface antigen. Its composition originates from the single chain variable domain (scFv) of the monoclonal antibody, and is composed of the heavy chain variable region (V_{H}) and light chain variable region (V_{L}). Intracellular signal transduction region is mainly composed of costimulatory signal and CD3zeta chain of T cell receptor (TCR). After CAR-expressing T (CAR-T) cells bind to target cell antigen through scFv, the intracellular signal transduction region transmits signals to T cells, thereby activating T cells, secreting perforins, granzymes and cytokines, etc., and exerting killing effects. At present, FDA has approved two CAR-T cell drugs, namely Kymriah of Novartis and Yescarta of Gilead. Both drugs target CD19-positive B-cell tumors and use the same antigen binding region, which is the scFv derived from the mouse monoclonal antibody FMC63.

The construction of CAR-T cells requires the transfection of CAR genes through viral or non-viral systems and integration into the T cell genome. When the gene is normally expressed and a transmembrane CAR structure is formed on the cell membrane, CAR-T cells have the activity of recognizing and killing target cells. Therefore, accurate detection of CAR-expressing positive T cells is a key step in CAR-T drug quality control, and is also an important link in clinical patient treatment dose control, process monitoring and auxiliary diagnosis. There are two common detection methods: detecting CAR gene positive T cells and detecting CAR protein positive T cells.

Detection of CAR gene positive T cells: Quantitative real time PCR (qPCR) is the main method to detect CAR gene positive T cells. The integrated CAR gene in cell genome and the copy number of CAR gene can be detected by qPCR. However, this method cannot accurately reflect the positive T cells expressing CAR, because some T cells integrated with CAR gene do not normally express CAR (Jennifer N. Brudno, et al., Journal of Clinical Oncology, 2016), false positives caused by these T cells reduce the accuracy of qPCR method application. More seriously, the qPCR method cannot distinguish CAR-positive T cells from CAR-positive B cells, which makes it difficult to find the risk of treatment and recurrence caused by CAR-positive B cells (Marco Ruella, et al., Nature Medicine, 2018). In addition, qPCR method cannot meet the demand of simultaneous detection of multiple surface markers (CD3, CD4, CD8, etc.) of T cells, which further limits the application of qPCR method.

Detection of CAR protein positive T cells: existing detection reagents include anti-mouse IgG (Fab ')₂ (Jackson ImmunoResearch, Inc.), Protein L (Zhili Zheng, et al., Journal of Translational Medicine, 2012), CD19/Fc (Satiro N De Oliveira, et al., Journal of Translational Medicine, 2013), GFP-CD19 (CN105859891), murine monoclonal antibody 136.20.1 (WO 2014/190273; Bipulendu Jena, et al., PLoS One, 2013). Among them, anti-mouse IgG (Fab ')₂ and protein L was not sensitive to CD19 CAR, and could not distinguish different CAR (such as CD22 CAR, BCMA CAR, etc.) constructed by different mouse original scFv. Both CD19/Fc and GFP-CD19 depend on the binding of CD19 protein to FMC63 scFv on CD19 CAR, because the affinity of scFv to CD19 is significantly lower than that of intact antibody FMC63 to CD19 (IAN C. Nicholson, et al., Molecular Immunology, 1997*),* so the sensitivity of CD19/Fc and GFP-CD19 in detecting CD19 CAR is lower. The detection of mouse original CD19 CARmonoclonal antibody 136.20.1 depends on the binding of the antibody to FMC63 scFv on CD19 CAR, and the detection sensitivity is close to 1: 1000, that is, one positive cell can be detected in 1000 cells. Its application in the detection of CD19 CAR positive cell *in vivo* still has the defect of low sensitivity. All of the above reagents have a small range of applications due to their own insurmountable reasons.

Therefore, it is urgent to develop CAR positive cell detection reagents and detection methods with high detection sensitivity and accuracy in this field to meet the requirements of CAR-T drug quality control, clinical treatment and auxiliary diagnosis.

### Summary of the invention

The invention is defined by the claims and any other aspects, configurations, instances or embodiments set forth herein not falling within the scope of the claims are for information only. Any references in the description to methods of treatment (or diagnosis) refer to the compounds, pharmaceutical compositions and medicaments of the present invention for use in a method of treatment of the human (or animal) body by therapy (or for diagnosis). The purpose of the present invention is to provide a CAR positive cell detection reagent with high detection sensitivity and high accuracy and application thereof.

In the first aspect of the present invention, there is provided an antibody having:
(1) a heavy chain variable region of an antibody comprising the following three complementarity determining regions or CDRs:
   CDR1 as shown in SEQ ID NO: 3: GIDFRNYG (SEQ ID NO: 3);
   CDR2 as shown in SEQ ID NO: 4: FSSSGST (SEQ ID NO: 4); and
   CDR3 as shown in SEQ ID NO: 5: ARHPGPTNGWKL (SEQ ID NO: 5);
      and
   a light chain variable region of an antibody having a complementarity determining regions or CDRs selected from the group consisting of:
      CDR1' as shown in SEQ ID NO: 6: QSVSGY (SEQ ID NO: 6);
      CDR2' as shown in SEQ ID NO: 7: RAS (SEQ ID NO: 7); and
      CDR3' as shown in SEQ ID NO: 8: QSNYNSGSSSSAAA (SEQ ID NO: 8);
      wherein the antibody is an antibody specific against CD19 antibody, and the CD19 antibody is a FMC63 scFv.

In another preferred embodiment, the heavy chain variable region has the amino acid sequence as shown in SEQ ID NO: 9.

QSLEESGGRLVTPGTPLTLTCTVSGIDFRNYGVSWVRQAPGKGLEWIGI
FSSSGSTYYATWAKGRFTISKASSTTVDLKMTSLTTEDTATYFCARHPGPTN
GWKLWGPGTLVTVSS (SEQ ID NO: 9)

In another preferred embodiment, the light chain variable region has the amino acid sequence shown in SEQ ID NO: 10.

DVVMTQTPASVEAPVGGTVTIKCQASQSVSGYCSWYQQKPGQPPKLLI
YRASTLESGVPSRFSGSGSGTDFTLTISDLECADAATYYCQSNYNSGSSSSAA
AFGGGTEVVVK (SEQ ID NO: 10)

In another preferred embodiment, the antibody has: a heavy chain; and/or a light chain.

In another preferred embodiment, the heavy chain constant region is of rabbit origin, mouse origin or human origin.

In another preferred embodiment, the light chain has the abovementioned light chain variable region and a light chain constant region.

In another preferred embodiment, the light chain constant region is of rabbit origin, mouse origin or human origin.

In another preferred embodiment, the antibody comprises a single chain antibody (scFv), a Fab or (Fab ')₂ fragment, a double-chain antibody, a monoclonal antibody.

In another preferred embodiment, the antibody is a rabbit-derived antibody.

In another preferred embodiment, the equilibrium dissociation constant K_{D} of the antibody with the scFv of the CD19 antibody is≤ 1 nM, more preferably, K_{D}≤ 100 pM, most preferably, K_{D}≤ 50 pM.

In another preferred embodiment, the scFv of the CD19 antibody is present on the cell membrane of CAR-T cells in the form of CAR.

In another preferred embodiment, the trade names of the CAR-T cells are Kymriah and Yescarta.

In the second aspect of the present invention, there is provided a recombinant protein having:
(i) the antibody according to the first aspect of the invention; and
(ii) an optional tag sequence assisting expression and/or purification.

In another preferred embodiment, the tag sequence comprises a secretory peptide tag.

In another preferred embodiment, the tag sequence comprises a 6His tag and/or an Fc tag.

In another preferred embodiment, the recombinant protein is specific to CD19 antibody.

The present disclosure concernsa polynucleotide encoding a polypeptide selected from the group consisting of the antibody according to the first aspect of the invention; or
(2) the recombinant protein according to the second aspect of the present invention.

The present disclosure also concerns a vector comprising the polynucleotide as above-disclosed.

In another preferred embodiment, the vectors include bacterial plasmids, bacteriophages, yeast plasmids, plant cell viruses, mammalian cell viruses (such as adenoviruses, retroviruses, lentiviruses, etc.), transposons or other vectors.

The present disclosure concernsa genetically engineered host cell comprising the vector as above-disclosed or having the polynucleotide as above-dislcosed integrated in the genome.

In the third aspect of the present invention, there is provided an immunoconjugate comprising:
(a) the antibody according to the fifth aspect of the invention; and
(b) a coupling moiety selected from the group consisting of a detectable label, a drug, a toxin, a cytokine, a radionuclide, an enzyme, a magnetic bead or agarose.

In another preferred embodiment, the coupling moiety is selected from the group consisting of: a fluorescent or a luminescent label, a radioactive label, MRI (magnetic resonance imaging) or CT (electronic computer X-ray tomography technique) contrast agent, or an enzyme capable of producing detectable products, a radionuclide, a biotoxin, a gold nanoparticle/nanorod, a nanoparticle of any form, or a particle of any form in other size (e.g. a magnetic bead, an agarose particle), etc.

In the fourth aspect of the present invention, there is provided a detection **reagent** for detecting CD19 CAR positive cells, comprising:
a first detection reagent which is the first antibody according to the first aspect of the present invention.

**In** another preferred embodiment, the detection reagent further comprises a second detection reagent specifically binding to the first antibody or a label coupled to the first antibody.

**In** another preferred embodiment, the first antibody is a monoclonal antibody coupled to a fluorescent or luminescent label.

And the detection sensitivity of the detection reagent is ≥ 1 CD19 CAR positive cell/1000 cells.

**In** another preferred embodiment, the first antibody is an unmodified monoclonal antibody.

**In** another preferred embodiment, the second detection reagent is a second antibody specific to the first antibody.

**In** another preferred embodiment, the first antibody is a biotinylated monoclonal antibody and the second detection reagent is a streptavidin with a detectable label.

**In** another preferred embodiment, the detection sensitivity of the detection reagent is ≥ 1 CD19 CAR positive cells/1000 cells, more preferably ≥ 5 CD19 CAR positive cells/10000 cells, more preferably ≥ 1 CD19 CAR positive cells/10000 cells, more preferably ≥ 2 CD19 CAR positive cells/100000 cells.

In another preferred embodiment, the monoclonal antibody is a rabbit-derived monoclonal antibody.

In another preferred embodiment, the first antibody and the second antibody are from different mammalian species.

In another preferred embodiment, the first antibody is of rabbit origin and the second antibody is of donkey or sheep origin.

In another preferred embodiment, the CAR positive cells include CAR immune cells and CAR non-immune cells.

In another preferred embodiment, the CAR positive cells are selected from the group consisting of T cells, NK cells, CIK cells, NKT cells, and combinations thereof.

**In** another preferred embodiment, the CAR positive cells include HEK-293T cells, Jurkat cells, and combinations thereof.

**In** another preferred embodiment, the monoclonal antibody further has one or more of the following characteristics:
(i) binding to the CD19 CAR scFv recombinant protein with EC₅₀ of 0.3-3 nM, preferably 0.3-1 nM;
(ii) binding to the CD19 CAR scFv recombinant protein with an effective concentration of 0.02-0.2 µg/ml, preferably 0.02-0.1 µg/ml;
(iii) the equilibrium dissociation constant with the CD19 CAR scFv recombinant protein is 10⁻¹⁰-10⁻¹²M.

In another preferred embodiment, the monoclonal antibody specifically recognizes and binds to CD19 CAR constructed based on scFv of anti-CD19 monoclonal antibody FMC63.

In another preferred embodiment, the extracellular antigen binding domain of the CD19 CAR has the amino acid sequence shown in SEQ ID NO: 1:

DIQMTQTTSSLSASLGDRVTISCRASQDISKYLNWYQQKPDGTVKLLIY
HTSRLHSGVPSRFSGSGSGTDYSLTISNLEQEDIATYFCQQGNTLPYTFGGGT
KLEITGGGGSGGGGSGGGGSEVKLQESGPGLVAPSQSLSVTCTVSGVSLPDY
GVSWIRQPPRKGLEWLGVIWGSETTYYNSALKSRLTIIKDNSKSQVFLKMNS
LQTDDTAIYYCAKHYYYGGSYAMDYWGQGTSVTVSS(SEQ ID NO: 1)

In another preferred embodiment, the CD19 CAR scFv recombinant protein has a CD19 CAR scFv-His tag structure.

In another preferred embodiment, the CD19 CAR scFv recombinant protein has the amino acid sequence of SEQ ID NO: 2:

DIQMTQTTSSLSASLGDRVTISCRASQDISKYLNWYQQKPDGTVKLLIY
HTSRLHSGVPSRFSGSGSGTDYSLTISNLEQEDIATYFCQQGNTLPYTFGGGT
KLEITGGGGSGGGGSGGGGSEVKLQESGPGLVAPSQSLSVTCTVSGVSLPDY
GVSWIRQPPRKGLEWLGVIWGSETTYYNSALKSRLTIIKDNSKSQVFLKMNS
LQTDDTAIYYCAKHYYYGGSYAMDYWGQGTSVTVSSHHHHHH (SEQ ID
NO: 2).

**In** another preferred embodiment, the monoclonal antibody is prepared by the following method:
(a) providing CD19 CAR scFv recombinant proteins; and
(b) immunizing rabbits with the recombinant proteins to obtain antiserum against the CD19 CAR scFv recombinant protein; and
(c) carrying out monoclonal culture with peripheral blood B cells of the immunized rabbits, screening positive B cell monoclones, thereby obtaining rabbit B cell monoclones against the CD19 CAR scFv recombinant protein; and
(d) sequencing the positive rabbit B cell monoclone to obtain the light/heavy chain gene sequence of the monoclonal antibody; and
(e) using the light/heavy chain gene sequence of the monoclonal antibody to construct expression plasmids and transfecting eukaryotic host cells to obtain transfected eukaryotic host cells; and
(f) culturing the transfected eukaryotic host cells under conditions suitable for expression to express the monoclonal antibody; and
(g) isolating and/or purifying the monoclonal antibody from the culture system.

In another preferred embodiment, the expression comprises secretory expression (i.e. secreted into the cell culture medium).

In another preferred embodiment, the eukaryotic host cells are selected from the group consisting of HEK-293T cells, CHO cells, COS-1 cells, and COS-7 cells.

In another preferred embodiment, the CD19 CAR scFv recombinant protein has the amino acid sequence of SEQ ID NO: 1 or 2.

In another preferred embodiment, the CD19 CAR scFv recombinant protein comprises a eukaryotic or prokaryotic expressed recombinant protein.

In another preferred embodiment, the CD19 CAR scFv recombinant protein is a recombinant protein expressed by *Escherichia coli.*

In another preferred embodiment, the CD19 CAR scFv recombinant protein is renatured.

In another preferred embodiment, the CD19 CAR scFv recombinant protein is eukaryotic expressed.

In another preferred embodiment, the CD19 CAR scFv recombinant protein is prepared by the following method:
(a) providing an expression plasmid comprising an expression cassette of the CD19 CAR scFv recombinant protein;
(b) transfecting the eukaryotic host cell with the expression plasmid, thereby obtaining the transfected eukaryotic host cell;
(c) culturing the transfected eukaryotic host cell under conditions suitable for expression, thereby expressing the recombinant protein; and
(d) isolating and/or purifying the recombinant protein from the culture system.

In another preferred embodiment, the expression comprises secretory expression (i.e. secreted into the cell culture medium).

In another preferred embodiment, the eukaryotic host cells are selected from the group consisting of HEK-293T cells, CHO cells, COS-1 cells, and COS-7 cells.

In the fifth aspect of the present invention, there is provided a CD19 CAR detection kit comprising:
a first container containing the antibody according to the first aspect of the present invention,
and optional instructions.

In another preferred embodiment, the antibody carries a detectable label.

In another preferred embodiment, the detectable label comprises a fluorescent or luminescent label.

In another preferred embodiment, the antibody is a fluorescently labeled monoclonal antibody.

In another preferred embodiment, the detection kit further comprises a second container, containing a secondary antibody specific to the antibody of the a first container, i.e. the antibody according to the first aspect of the present invention.

In another preferred embodiment, the first container and the second container may be integral or separate.

In another preferred embodiment, the second antibody is an antibody against rabbit IgG.

In another preferred embodiment, the second antibody is selected from the group consisting of a donkey anti-rabbit IgG antibody and a goat anti-rabbit IgG antibody.

In another preferred embodiment, the second antibody carries a detectable label.

In another preferred embodiment, the detectable label comprises a fluorescent or luminescent label.

In another preferred embodiment, the second antibody is a fluorescently labeled secondary antibody.

In another preferred embodiment, the detection kit further comprises one or more additional detection reagents selected from the group consisting of:
a third container, and a sealing agent within the third container; and
a fourth container, and a positive control (e.g. a CD19 CAR scFv recombinant protein) within the fourth container; and
a fifth container, and a negative control reagent within the fifth container.

In another preferred embodiment, the additional detection reagents are located in the same or different containers.

In another preferred embodiment, the instructions describe a method for detecting CD19 CAR positive cells.

In the sixth aspect of the present invention, there is provided a use of the detection reagent according to the fourth aspect of the present invention for preparing a reagent or kit for detecting CD19 CAR positive cells *in vitro.*

In another preferred embodiment, the CAR positive cells include CAR immune cells and CAR non-immune cells.

In another preferred embodiment, the CAR positive cells are selected from the group consisting of T cells, NK cells, CIK cells, NKT cells, and combinations thereof.

In another preferred embodiment, the CAR positive cells include HEK-293T cells, Jurkat cells, and combinations thereof.

In the seventh aspect of the present invention, there is provided an *in vitro* method for detecting CD19 CAR positive cells, comprising the steps of:
(I) providing the detection reagent according to the fourth aspect of the present invention;
(II) detecting a cell population to be tested with the detection reagent, thereby obtaining a qualitative or quantitative detection result of CD19 CAR positive cells.

In another preferred embodiment, the method comprises the steps of:
(1) taking 1 × 10⁵-1 × 10⁶ cells to be tested, and washing 1-3 times with buffer containing blocking agent (such as PBS, containing 1% blocking agent);
(2) resuspending the cells with 50-200µL of a buffer containing blocking agent and monoclonal antibodies with detectable labels (e.g., PBS, containing 1% blocking agent, and 1-100ng of the monoclonal antibody), and placing at 2-15°C (preferably 4±2°C) for a period of time T1 (e.g., 10-60 minutes, preferably about 15-45 minutes);
(3) washing the cells 1-3 times with buffer (e.g. PBS);
(4) resuspending the cells with a certain amount (such as 100-500µL) of buffer (e.g. PBS) and detecting by flow cytometry.

In another preferred embodiment, the detectable label comprises a fluorescent or luminescent label.

In another preferred embodiment, the monoclonal antibody is a fluorescently labeled monoclonal antibody.

In another preferred embodiment, the method comprises the steps of:
(1) taking 1 × 10⁵-1 × 10⁶ cells to be tested, and washing 1-3 times with buffer containing blocking agent (such as PBS, containing 1% blocking agent);
(2) resuspending the cells with 50-200µL of a buffer containing blocking agent and the first antibodies (monoclonal antibodies) (e.g., PBS, containing 1% blocking agent, and 1-100ng of the monoclonal antibody such as R19mab), and placing at 2-15°C (preferably 4±2°C) for a period of time T1 (e.g., 10-60 minutes, preferably about 15-45 minutes);
(3) washing the cells 1-3 times with buffer (e.g. PBS);
(4) resuspending the cells with 50-200µL of a buffer containing the second antibodies with detectable labels (such as fluorescent groups) (e.g., PBS, containing 100-500ng of the secondary antibody), and placing at 2-15°C (preferably 4±2°C) for a period of time T2 (e.g., 10-60 minutes, preferably about 15-30 minutes);
(5) washing the cells 1-3 times with buffer (e.g. PBS);
(6) resuspending the cells with a certain amount (such as 100-500µL) of buffer (e.g. PBS) and detecting by flow cytometry.

In another preferred embodiment, the method is a non-diagnostic and non-therapeutic method.

In another preferred embodiment, the method is a quality control method.

It should be understood that within the scope of the present invention, each technical features of the present invention described above and in the following (as examples) may be combined with each other to form a new or preferred technical solution, which is not listed here due to space limitations.

### Description of Figure

Figure 1 shows the results of detecting the titer of CD19 CAR positive cells by rabbit antiserum of the present invention.
Figure 2 shows the results of detecting CD19 CAR positive cells by rabbit B cell monoclonal culture supernatant.
Figure 3 shows the ELISA results of the rabbit monoclonal antibody R19mab of the present invention binding to FMC63 scFv and FMC63, compared with the data of mouse monoclonal antibody 136.20.1.
Figure 4 shows the results of the detection of different target CAR-T (CD19 CAR-T and BCMA CAR-T) cells by the rabbit monoclonal antibody R19mab of the present invention.
Figure 5 shows the results of FMC63 scFv blocking the binding of R19mab to CD19 CAR-T. In which Figure 5A shows the blocking effect of different concentrations of FMC63 scFv, and Figure 5B shows the IC₅₀ measurement results of the blocking effect of FMC63 scFv.
Figure 6 shows the EC₅₀ measurement results of R19mab binding to CD19 CAR scFv on the cell surface.
Figure 7 shows the results of the sensitivity assay of R19mab binding to CD19 CAR positive cells, compared with the data of mouse monoclonal antibody 136.20.1 and the data of CD19/Fc.
Figure 8 shows the results of immunofluorescence assay of R19mab binding to CD19 CAR scFv on the cell surface.
Figure 9 shows the results of kinetic analysis of R19mab binding to FMC63 scFv.

### Detailed Description

Through extensive and intensive research and a lot of screening, the inventors have unexpectedly obtained a monoclonal antibody R19mab against CD19 antibody. Experimental results show that the monoclonal antibody against CD19 antibody has high specificity and affinity. The present invention also provides a detection reagent with high sensitivity, good accuracy and good specificity for the detection of CAR-T cells. The detection method based on the detection reagent of the present invention can not only directly target the extracellular antigen binding region on CAR-T cells, but also has a detection sensitivity much higher than that of various existing detection reagents. In addition, the detection reagent of the present invention has a very high degree of discrimination between positive CAR-T cells and negative CAR-T cells, and the detection result is more accurate. The present invention has been completed on this basis.

### Term

As used herein, the term "antibody" or "immunoglobulin" is a heterotetrameric glycoprotein of about 150,000 Da having the same structural characteristics, which consists of two identical light chains (L) and two identical heavy chains (H). Each light chain is linked to a heavy chain via a covalent disulfide bond, and different immunoglobulin isotypes have different numbers of disulfide bonds between the heavy chains. There are also regularly spaced intrachain disulfide bonds in each heavy and each light chain. Each heavy chain has a variable region (VH) at one end, followed by a plurality of constant regions. Each light chain has a variable region (VL) at one end and a constant region at the other end; the constant region of light chain pairs with the first constant region of heavy chain, and the variable region of light chain pairs with the variable region of heavy chain. Special amino acid residues form an interface between the variable regions of a light chain and a heavy chain.

As used herein, the term "variable" means that certain portion of the variable region in an antibody differ in sequence, which is responsible for the binding and specificity of various specific antibodies to their specific antigen. However, the variability is not distributed evenly throughout the variable regions of an antibody. It is concentrated in three fragments called complementarity determination regions (CDRs) or hypervariable regions in light chain and heavy chain variable regions. The conserved parts of variable regions are called framework regions (FRs). Each of the variable regions of naturally occurring heavy and light chains comprises four FR regions, which are generally in a β-sheet configuration, joined by the three CDRs forming a linking loop, and in some cases, may form a partial β-sheet structure. The CDRs in each chain are closely linked together via the FR regions, and together with the CDRs of the other chain, form the antigen binding site of an antibody (see Kabat et al., NIH Publ. No. 91-3242, Volume I, pages 647-669 (1991)). Constant regions are not directly involved in the binding of antibodies to antigen, however, they exhibit different effector functions, such as participating in the antibody-dependent cytotoxicity of antibodies.

As used herein, the term "monoclonal antibody (mAb)" refers to an antibody obtained from a substantially homogeneous population, i.e., the individual antibodies contained in the population are the same, except for a few naturally occurring mutations that may be present. A monoclonal antibody is highly specific for a single antigenic site. Moreover, unlike conventional polyclonal antibody preparations (usually with different antibodies directed against different determinants), each monoclonal antibody is directed against a single determinant on the antigen. In addition to their specificity, the advantage of monoclonal antibodies is that they can be synthesized by recombinant expression of eukaryotic cells and will not be contaminated by other immunoglobulins. The modifier "monoclonal" indicates the characteristics of antibodies, which are obtained from a substantially uniform antibody population, which should not be interpreted as requiring any special method to produce antibodies.

The present invention includes not only complete monoclonal antibodies, but also antibody fragments with immune activity, such as single chain antibodies; Fab or Fab '₂ fragments; antibody heavy chains; antibody light chains.

As used herein, the terms "heavy chain variable region" and "V_{H}" can be used interchangeably.

As used herein, the terms "variable region" and "complementarity determine region (CDR)" can be used interchangeably.

In a preferred embodiment of the present invention, the heavy chain variable region of the antibody comprises the following three complementarity determining regions or CDRs:
CDR1, whose amino acid sequence is GIDFRNYG (SEQ ID NO: 3);
CDR2, whose amino acid sequence is FSSSGST (SEQ ID NO: 4);
CDR3, whose amino acid sequence is ARHPGPTNGWKL (SEQ ID NO: 5).

In another preferred embodiment, the amino acid sequence of the heavy chain variable region is:

QSLEESGGRLVTPGTPLTLTCTVSGIDFRNYGVSWVRQAPGKGLEWIGI
FSSSGSTYYATWAKGRFTISKASSTTVDLKMTSLTTEDTATYFCARHPGPTN
GWKLWGPGTLVTVSS (SEQ ID NO: 9).

In a preferred embodiment of the present invention, the heavy chain of the antibody comprises the aforementioned heavy chain variable region and a heavy chain constant region, which may be of rabbit origin, mouse origin or human origin.

As used herein, the terms "light chain variable region" and "V_{L}" can be used interchangeably.

In a preferred embodiment of the invention, the light chain variable region of the antibody according to the invention has complementarity determining regions CDR selected from the group consisting of:
CDR1', whose amino acid sequence is QSVSGY (SEQ ID NO: 6);
CDR2', whose amino acid sequence is RAS (SEQ ID NO: 7);
CDR3', whose amino acid sequence is QSNYNSGSSSSAAA (SEQ ID NO: 8).

In another preferred embodiment, the amino acid sequence of the light chain variable region is:

DVVMTQTPASVEAPVGGTVTIKCQASQSVSGYCSWYQQKPGQPPKLLI

YRASTLESGVPSRFSGSGSGTDFTLTISDLECADAATYYCQSNYNSGSSSSAA
AFGGGTEVVVK (SEQ ID NO: 10).

The invention also provides other proteins or fusion expression products having antibodies of the invention. Specifically, the present invention includes any protein or protein conjugate and fusion expression product (i.e., immunoconjugate and fusion expression product) having a heavy chain and a light chain containing variable regions, as long as the variable region is the same as or has at least 90% homology with the variable regions of the heavy chain and light chain of the antibody of the present invention, preferably at least 95% homology.

As used herein, the term "CAR" refers to the chimeric antigen receptor, comprising an extracellular domain, a transmembrane domain, and an intracellular domain. The extracellular domain includes a target-specific binding element (also called antigen binding domain). The intracellular domain includes a costimulatory signal transduction region and a zeta chain part. The costimulatory signal transduction region refers to a part of intracellular domain including costimulatory molecules. The costimulatory molecules are cell surface molecules needed by lymphocytes to effectively respond to antigens.

### Kit

The present invention also provides a detection kit for detecting CD19 CAR. The detection kit (direct labeling method) comprises:
(t1) a first container, and monoclonal antibodies with detectable labels within the first container, and the monoclonal antibody specifically binds to the extracellular antigen binding domain of CD19 CAR.
(t0) instructions.

In another preferred embodiment, the detectable label comprises a fluorescent or luminescent label.

In another preferred embodiment, the monoclonal antibody is a fluorescently labeled monoclonal antibody.

In another preferred embodiment, the detection kit (secondary antibody method) comprises:
(t1) a first container, and a first antibody within the first container, wherein the first antibody is a monoclonal antibody specifically binding to the extracellular antigen binding domain of CD19 CAR; and
(t2) a second container, and a second antibody within the second container that is specific to the monoclonal antibody; and
(t0) instructions.

In another preferred embodiment, the second antibody is an antibody against rabbit IgG.

In another preferred embodiment, the second antibody is selected from the group consisting of a donkey anti-rabbit IgG antibody and a goat anti-rabbit IgG antibody.

In another preferred embodiment, the second antibody carries a detectable label.

In another preferred embodiment, the detectable label comprises a fluorescent or luminescent label.

In another preferred embodiment, the second antibody is a fluorescently labeled secondary antibody.

In another preferred embodiment, the detection kit further comprises one or more additional detection reagents selected from the group consisting of:
(t3) a third container, and a sealing agent within the third container; and
(t4) a fourth container, and a positive control (e.g. a CD19 CAR scFv recombinant protein) within the fourth container; and
(t5) a fifth container, and a negative control reagent within the fifth container.

In another preferred embodiment, the additional detection reagents are located in the same or different containers.

In another preferred embodiment, the instructions describe a method for detecting CD19 CAR positive cells.

### Detection method

The present invention also provides an *in vitro* method for detecting CAR positive cells based on the monoclonal antibody of the invention. Since the monoclonal antibody of the present invention has high affinity and high specificity for the antigen binding region of matched or corresponding CAR, the monoclonal antibody of the present invention can be used to efficiently, sensitively and accurately detect CAR positive cells.

For the CAR positive cells that can be detected by the method of the present invention, representative examples include, but are not limited to: T cells, NK cells, NKT cells, CIK cells and many other different cells. For example, taking the rabbit monoclonal antibody (R19 mab) against FMC63 scFv of the present invention as an example, it can be used for the detection of corresponding CD19 CAR positive cells (including T, NK, NKT or CIK cells).

The detection method of the present invention can be used in scientific research, research and development of cell drugs, quality control of cell drugs, clinical treatment monitoring of cell drugs, auxiliary diagnosis of clinical patients and the like.

The main advantages of the present invention include:
(a) The equilibrium dissociation constant K_{D} value of the rabbit monoclonal antibody R19mab of the present invention binding to the FMC63 scFv reaches 43 pM, and the detection sensitivity of R19mab determined by flow cytometry reaches 1: 10000. Therefore, the detection kit for CAR positive cells developed based on the monoclonal antibody of the present invention has high detection sensitivity and good specificity.
(b) The detection reagent and method of the present invention have a very high degree of discrimination between CAR positive cells and CAR negative cells, and the detection results are more accurate.
(c) The rabbit monoclonal antibody of the present invention has a high affinity to mouse FMC63 (significantly better than the affinity of mouse monoclonal antibody to mouse FMC63).
(d) Compared with existing detection reagents such as anti-mouse IgG (Fab')₂, Protein L, CD19/Fc, GFP-CD19, mouse monoclonal antibody 136.20.1, the rabbit monoclonal antibody of the present invention can detect CD19 CAR positive cells more specifically, sensitively and accurately, and meet the requirements of CAR-T drug production quality control, clinical treatment monitoring and auxiliary diagnosis.
(e) The production cost of the rabbit monoclonal antibody and the kit of the present invention is significantly reduced, so it is more advantageous to be applied to CAR-T drug quality control, clinical treatment monitoring and auxiliary diagnosis.

The present invention is further explained below in conjunction with specific example. It should be understood that these examples are only for illustrating the present invention and not intend to limit the scope of the present invention. The conditions of the experimental methods not specifically indicated in the following examples are usually in accordance with conventional conditions as described in Sambrook et al., Molecular Cloning: A Laboratory Manual (New York: Cold Spring Harbor Laboratory Press, 1989), or according to the conditions recommended by the manufacturer. Unless otherwise stated, percentages and parts are percentages by weight and parts by weight.

### General methods

1. T cell activation and CD19 CAR lentivirus infection
   (1) 0.5 × 10⁶ T cells (0.5 × 10⁶/mL) were taken, and placed in a 24-well plate.
   (2) The magnetic beads coated with anti-CD3/CD28 were washed with R10 culture medium 3 times according to the instructions.
   (3) Magnetic beads were added to T cells at a ratio of 3: 1 (magnetic beads: cells), and placed in an incubator at 37 °C, 5% CO₂ overnight.
   (4) After activating T cells for 12-24 hours, the 24-well plate was centrifuged to remove 800µL supernatant culture medium.
   (5) Lentivirus particles were thawed at room temperature, and mixed gently. 1mL lentivirus particles was added into 0.5 × 10⁶ T cells (200L), and polybrene was added to a final concentration of 8g/mL, then placed in a centrifuge at 2500 rpm for 1.5 hours, and placed in an incubator after centrifugation at 37 °C, 5% CO₂ overnight.
   (6) The 24-well plate cultured overnight was centrifuged to remove most supernatant culture medium containing lentivirus particles. Fresh R10 culture medium was added to expand T cells.
   (7) T cells were counted every 2 days, and IL-2 50IU/mL was added to maintain T cells at 0.5-1 × 10⁶/ml.
   (8) After 3-5 days, 2 × 10⁵ cells were taken to detect CD19 CAR-T cells by flow cytometry.
2. Detection of CD19 CAR-T cells by flow cytometry (secondary antibody method)
   (1) 2 × 10⁵ CD19 CAR-T cells were taken, washed twice with PBS and resuspended with 100µL PBS.
   (2) Detection antibodies (or other detection reagents) with corresponding dilution ratio were added, and placed at 4 °C for 45 minutes.
   (3) Washed twice with PBS.
   (4) Fluorescent secondary antibody was added, and placed at 4 °C in the dark for 30 minutes.
   (5) Washed with PBS twice.
   (6) Resuspended with 200µL PBS and detected by flow cytometry.
3. Detection of CD19 CAR-T cells by flow cytometry (direct labeling method)
   (1) 2 × 10⁵ CD19 CAR-T cells were taken, washed twice with PBS and resuspended with 100µL PBS.
   (2) Fluorescent labeled detection antibodies (or other detection reagents) with corresponding dilution ratio were added, and placed at 4 °C for 45 minutes.
   (3) Washed twice with PBS.
   (4) Resuspended with 200µL PBS and detected by flow cytometry.

### EXAMPLE 1. Preparation Method of Rabbit Monoclonal Antibody (R19mab) Against FMC63 scFv

### (1) Preparation and purification of recombinant antigen

An expression plasmid containing the FMC63 scFv-His tag recombinant antigen sequence (amino acid sequence as shown in SEQ ID NO: 2) was constructed. 293T cells were cultured in a 10 cm culture dish. According to the instructions of lipofectamine, the expression plasmid containing the recombinant antigen sequence of FMC63 scFv-His tag was transfected into 293T cells. The culture medium was harvested on the sixth day after transfection, and the cells were removed by centrifugation to obtain supernatant. The recombinant antigen in the supernatant was purified according to the instructions of Ni-NTA Superflow of QIAGEN Company.

Results: After purification, the antigen concentration was 0.5-4 mg/ml.

### (2) Immunization of rabbits with recombinant antigen

The purified recombinant protein was used as antigen to immunize rabbits. In short, 0.5 mL of Freund's complete adjuvant was fully mixed with 0.5 mL of antigen (200 µg). Each rabbit was immunized 4 parts (both the back and thigh root), and each part was 0.25 mL. The immunization cycle was 20 days, and blood was taken 7-10 days after immunization to detect antiserum titer. A total of 4-5 immunizations were conducted.

### (3) Detection of CD19 CAR positive cell titer by rabbit antiserum

**The lentiviral plasmid containing FMC63 scFv CD19 CAR sequence and** the lentiviral plasmid containing BCMA CAR sequence were mixed with three packaging plasmids VSVg, gag/pol, rev, respectively, and 293T cells were transfected with lipofectamine reagent. 24 and 48 hours after transfection, cell supernatant was collected, filtered and ultracentrifuged to obtain purified CD19 CAR lentivirus and BCMA CAR lentivirus, and stored at -80 °C. CD19 CAR-293T cells were obtained by infecting 293T cells with CD19 CAR lentivirus. Rabbit antiserum was gradient diluted with cold FACS buffer. 2 × 10⁵ CD19 CAR-293T cells were suspended in 50 µL FACS buffer. 50 µL dilute solution of antiserum was then added to 50 µL cell suspension and the mixture was incubated on ice for 30 minute. The cells were then washed twice with cold FACS buffer. Goat anti-rabbit IgG (H + L)-Alexa Fluor 647 secondary antibody (Table 1) was added and the mixture was incubated on ice for 30 minutes, then the cells were washed twice with cold FACS buffer. The cells were resuspended in 200 µL FACS buffer and detected by flow cytometry.

### Results:

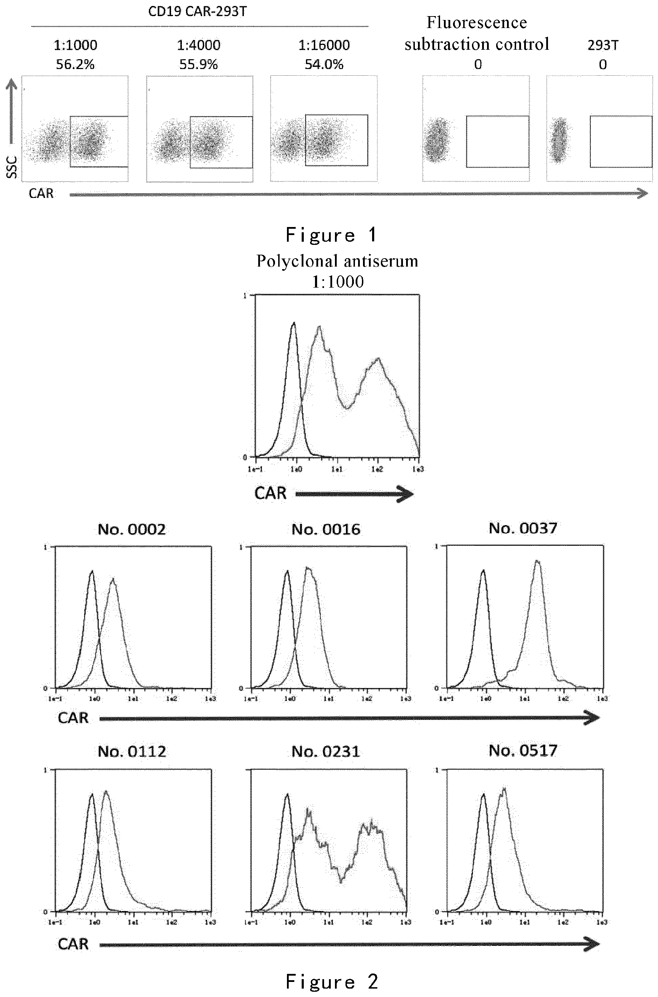

As shown in Figure 1, rabbit antiserum diluted at 1:16000 can detect CD19 CAR positive cells normally, indicating that the titer has reached at least ≥ 1:16000. The recombinant antigen prepared in Example 1 (1) can efficiently produce specific anti-FMC63 scFv antibodies in rabbits.

### (4) Monoclonal screening of rabbit B cells

Rabbit B cell monoclone expressing anti-FMC63 scFv antibodies was screened using the method of Stefan Seeber et al. (Stefan Seeber, et al., PLoS ONE, 2014). In short, peripheral blood of rabbits producing anti-FMC63 scFv antibodies was taken and peripheral blood mononuclear cells were isolated. FMC63 scFv recombinant protein was used to enrich positive B cell monoclones, and then B cell monoclones were cultured in 96-well plates. After 7 days, the B cell culture supernatant was taken, and flow cytometry test was performed according to the same protocol described in Example 1 (3), to identify rabbit B cell positive clones expressing anti-FMC63 scFv antibody. The positive clones were lysed and the heavy/light chain genes were obtained by RT-PCR. The heavy/light chain gene expression vector was constructed and transfected into 293T cells for expression. After 7 days, the cell culture supernatant was taken, and flow cytometry test was performed according to the same protocol described in Example 1 (3), to confirm the monoclonal antibody against FMC63 scFv, and then the heavy/light chain gene sequence of the antibody was obtained by sequencing.

### Results:

As shown in Figure 2, the B cell culture supernatant was screen by flow cytometry assay to confirm that No.0231 was a rabbit B cell positive clone expressing anti-FMC63 scFv antibody. The heavy/light chain gene sequence of the antibody was obtained by sequencing, and the antibody was R19mab.

### (5) Purification of rabbit monoclonal antibody R19mab

The culture supernatant of 293T cells expressing R19mab was loaded into Protein-A chromatography column at a flow rate of about 1 mL/min, 20 mL equilibrium buffer (20 mM Na₂HPO₄, 0.15 M NaCl, pH 7.0) was used to wash resin at a flow rate of about 2 mL/min. The antibody was eluted with 10 mL elution buffer (0.1 M Glycine, pH 3.0) at a flow rate of about 1 mL/min, the elution was collected, and a neutralization buffer (1 M Tris, pH 8.5) of 1/10 volume of the elution buffer was immediately added to adjust the pH to 7.4. Concentrated to 1-1.5 mL with ultrafiltration tube, and then the antibody concentration was determined by spectrophotometer.

Results: After purification, the concentration of R19mab antibody was 0.5-4 mg/ml.

### EXAMPLE 2. Structural Characterization of R19mab

R19mab comprises a heavy chain variable region and a light chain variable region, wherein the complementarity determining region of the heavy chain variable region comprises the amino acid sequence of CDR1 as shown in SEQ IDNO: 3, the amino acid sequence of CDR2 as shown in SEQ ID NO: 4 and the amino acid sequence of CDR3 as shown in SEQ ID NO: 5, and the complementarity determining region of the light chain variable region comprises the amino acid sequence of CDR1' as shown in SEQ ID NO: 6, the amino acid sequence of CDR2' as shown in SEQ ID NO: 7 and the amino acid sequence of CDR3' as shown in SEQ ID NO: 8.

### EXAMPLE 3. Characterization of Binding Properties of R19mab

In this example, the binding of R19mab to FMC63 and FMC63 scFv was detected by ELISA. The binding of R19mab to CD19 CAR on the cell surface was detected by flow cytometry and immunofluorescence. The binding kinetics of R19mab to FMC63 scFv was analyzed by Fortebio Octet.

### (1) ELISA analysis

The binding specificity of R19mab to FMC63 and FMC63 scFv was detected by ELISA. The method is as follows:
96-well titer plates were coated with PBS containing 1 µg/ml of purified FMC63 scFv recombinant protein, FMC63 antibody or murine IgG at 100 µL per well (100 ng was coated), and incubated overnight at 4°C. Blocking was then performed with PBST (PBS containing 0.05% Tween 20) containing 5% BSA at 300 µL per well. Different concentrations of R19mab (0.022-5.625 µg/ml) were added to each well and incubated at ambient temperature for 1 hour. The plate was washed with PBST and then incubated at room temperature for 30 minutes with goat anti-rabbit IgG polyclonal antibody coupled with horseradish peroxidase (HRP). After washing, the plate was developed with an ABTS substrate and analyzed by spectrophotometer at OD 405.

### Results:

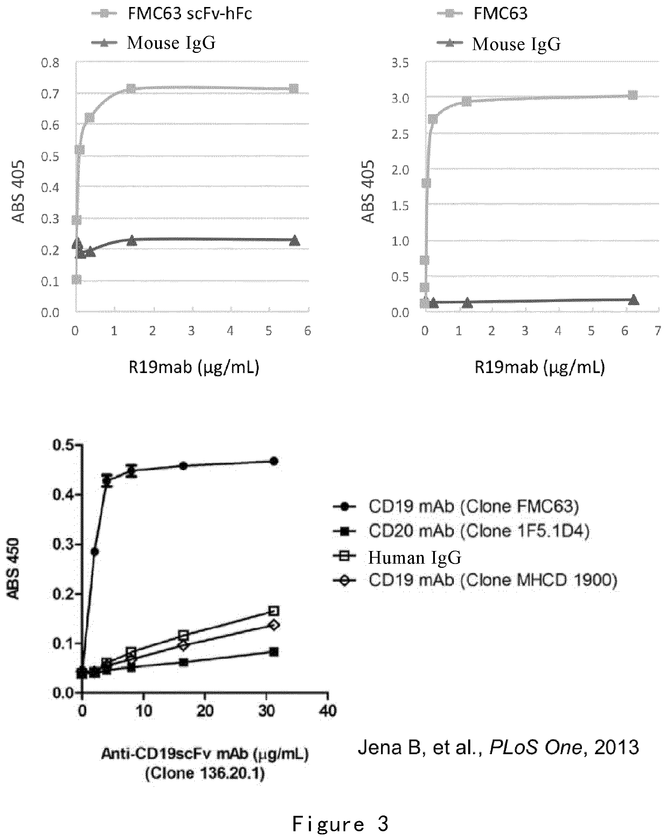

As shown in Figure 3, the result of ELISA showed that R19mab bound to FMC63 scFv and FMC63 antibody, but did not bind to murine IgG, indicating that R19mab specifically recognized FMC63 scFv and FMC63 antibody and did not cross-react to other murine IgG. R19mab binds to FMC63 scFv with an EC₅₀ of 0.307 nM. The effective binding concentration is 0.022 µg/mL, and the saturated binding concentration is 0.352 µg/mL. R19mab binds to FMC63 with an EC₅₀ of 0.253 nM. The effective binding concentration is 0.002 µg/mL, and the saturated binding concentration is 0.25 µg/mL. The effective binding concentration of the murine monoclonal antibody 136.20.1 invented by Cooper et al. for binding to FMC63 is 2 µg/mL, and the saturated binding concentration is 4 µg/mL. Compared with 136.20.1, R19mab has significant advantages.

Therefore, under similar conditions, the effective binding concentration of the antibody of the present invention can be as low as 0.002 µg/mL, while the effective binding concentration of the existing antibody is 2 µg/mL, and the difference between the two is about 1000 times. In addition, the saturated binding concentration of the antibody of the present invention is 0.25 µg/mL, which is about 125 times of the effective binding concentration, while the saturated binding concentration of the existing antibody is only 2 times of the effective binding concentration. Therefore, the detection range of the antibody of the present invention is large, thereby saving costs.

### (2) Flow cytometry research

### (I) CD19 CAR-T cell binding

T cells were infected with CD19 CAR lentivirus or BCMA CAR lentivirus to obtain corresponding CAR-T cells. R19mab, Protein L and isotype control (Rabbit IgG) antibody (Table 1) were diluted into 50 µL precooled FACS buffer. 2 × 10⁵ cells were suspended in 50 µL FACS buffer. 50 µL antibody solution was then added to 50 µL cell suspension and the mixture was incubated on ice for 30 minutes. The cells were then washed twice with cold FACS buffer. The corresponding secondary antibody (R19mab, Rabbit IgG/Goat anti-Rabbit IgG (H + L), Alexa Fluor 488; Protein L/Streptavidin, FITC) was added. Table 1) and the mixture was incubated on ice for 30 minutes, after that the cells were washed twice with cold FACS buffer. The cells were resuspended in 200 µL FACS buffer and detected by flow cytometry.

### Results:

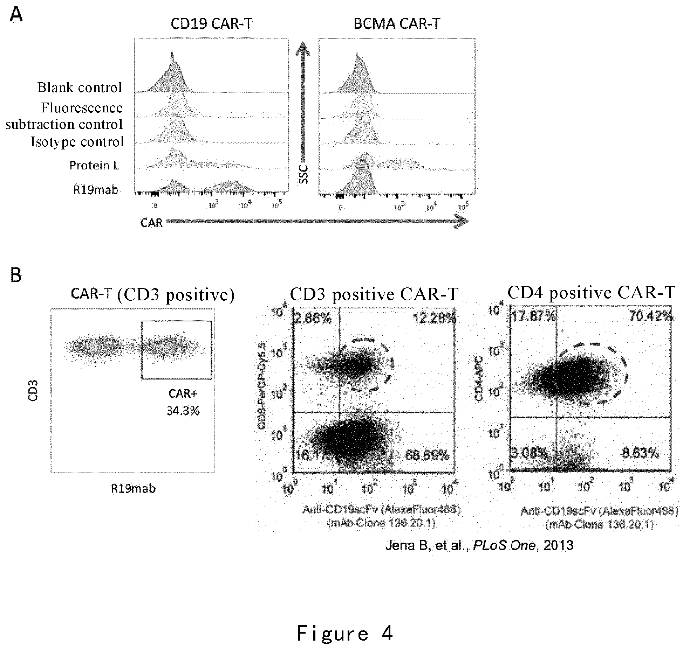

As shown in Figure 4A, R19mab recognizes the CD19 CAR structure on the cell surface, but not the BCMA CAR structure. The difference between the two is only in the scFv part. Therefore, R19mab specifically recognizes CD19 CAR positive cells containing FMC63 scFv sequences. On the contrary, Protein L cannot distinguish CD19 CAR from BCMA CAR, and the detection is not specific. In addition, R19mab can clearly distinguish CD19 CAR positive and negative cell groups, and the signal peaks of the two do not overlap. However, the negative signal and positive signal of Protein L detection results partially overlap, which makes it impossible to clearly distinguish positive and negative cell groups, and the detection accuracy is low.

As shown in Figure 4B, when CD19 CAR-T cells are detected by R19mab, the positive signal (box) is completely separate from the negative signal. When CD19 CAR-T cells are detected by mouse monoclonal antibody 136.20.1 invented by Cooper et al., neither CD4 positive nor CD8 positive CAR-T (box) can be completely separated from negative T cells.

Therefore, the rabbit monoclonal antibody R19mab of the present invention is far superior to Protein L and mouse monoclonal antibody 136.20.1 in terms of detection specificity and accuracy.

In addition, combined with the detection method specific for CD4 and/or CD8, the method of the present invention can further effectively distinguish two subsets of CAR-positive CD4-positive T cells and CD8-positive T cells.

Because R19mab has high specificity and accuracy, and can clearly distinguish CD19 CAR positive and negative cell groups, CD19 CAR positive cells can be counted conveniently and accurately, which is very important for quality control of CD19 CAR-T drug production and auxiliary diagnosis of clinical treatment.

### (II) FMC63 scFv blocking R19mab binding to CD19 CAR-T

The purified FMC63 scFv recombinant protein was gradient diluted with 1 × PBS, R19mab (final concentration was 0.1 µg/mL) was added to the diluent, and incubated on ice for 30 minutes. 2 × 10⁵ CD19 CAR-T cells were suspended in 100 µL FACS buffer. A mixed solution of FMC63 scFv/R19mab was then added to the cell suspension, and a flow cytometry experiment was performed according to the same protocol described in Example 3 (2) (I) to analyze the blocking effect of FMC63 scFv on R19mab binding to CD19 CAR-T.

### Results:

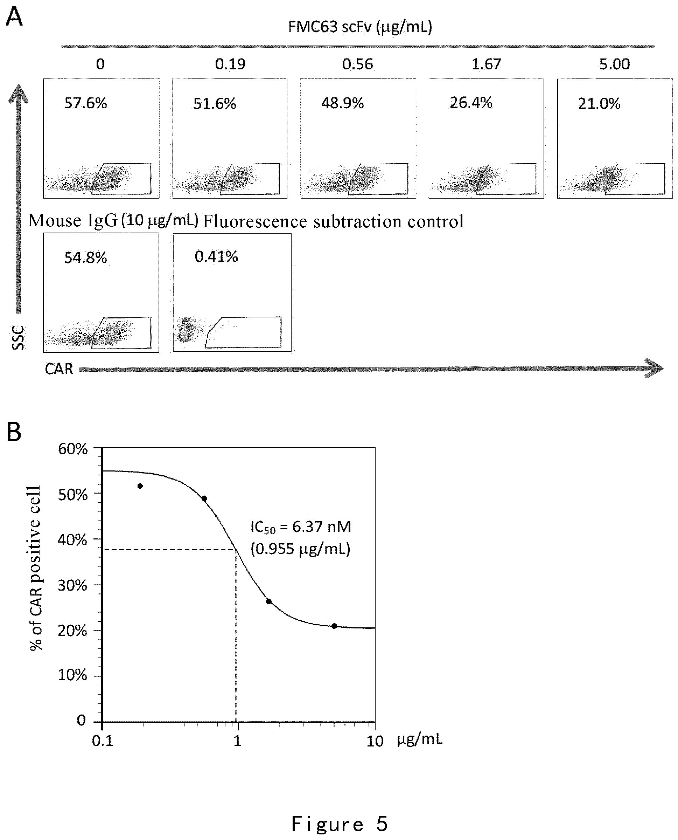

As shown in Figure 5, FMC63 scFv effectively block the binding of R19mab to CD19 CAR-T (Figure 5A), with an IC₅₀ of 6.37 nM (0.955 µg/mL) (Figure 5B). The results indicate that R19mab specifically recognizes FMC63 scFv fragment on CD19 CAR.

### (III) CD19 CAR-293T cell binding

CD19 CAR-293T cells were obtained by infecting 293T cells with CD19 CAR lentivirus. R19mab was gradient diluted with cold FACS buffer. 2 × 10⁵ CD19 CAR-293T cells were suspended in 50 µL FACS buffer. Then 50 µL R19mab antibody solution was added to 50 µL cell suspension, and flow cytometry was performed according to the same protocol described above to analyze R19mab binding.

### Results:

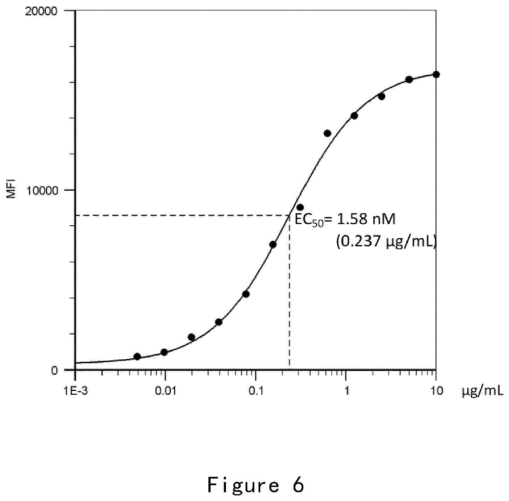

As shown in Figure 6, R19mab effectively binds to CD19 CAR scFv on the cell surface, with an EC₅₀ of 1.58 nM.

### (IV) Sensitivity analysis of R19mab detection

CD19 CAR-T cells were mixed with different numbers of PBMC cells to reach CAR-T: PBMC cell ratios ranging from 1:10 to 1:10, 000. Flow cytometry assays were performed according to the same protocol described in Example 1 (3) to analyze the sensitivity of R19mab in detecting CD19 CAR positive cells.

### Results:

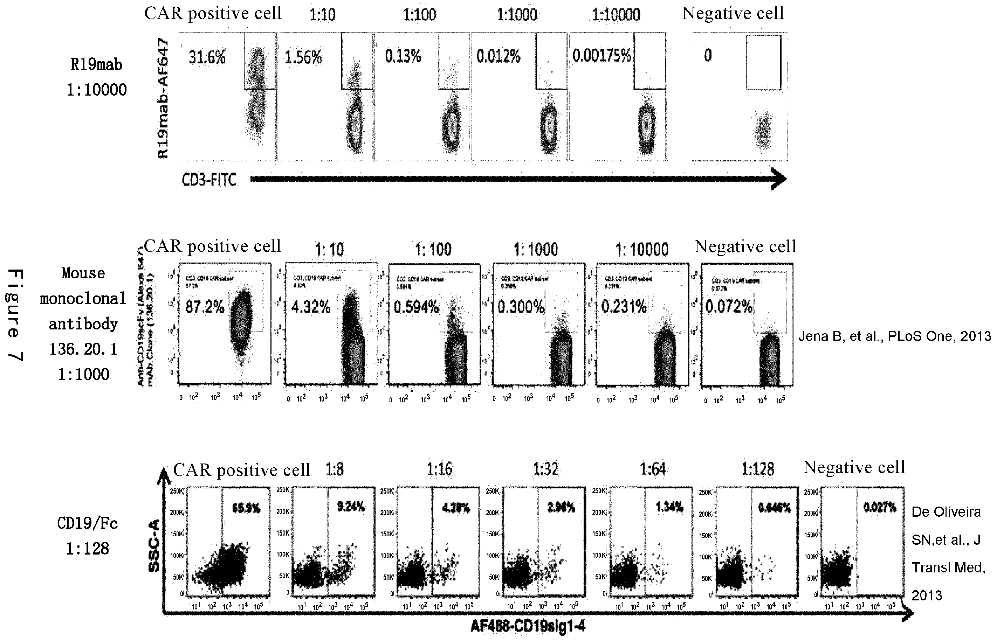

As shown in Figure 7, two CD19 CAR positive cell out of 100,000 negative cells were detected using R19mab, with a detection sensitivity of 0.002%. The murine monoclonal antibody 136.20.1 invented by Cooper et al. has a detection sensitivity of 0.1% (Bipulendu Jena, et al., PLoS One, 2013), and the detection sensitivity of CD19/Fc (the number in the article is AF488-CD19sIg1-4) reaches 0.5% (Satiro N De Oliveira, et al., Journal of Translational Medicine, 2013*).* Therefore, in terms of detection sensitivity, R19mab is far superior to murine monoclonal antibodies 136.20.1 and CD19/Fc. Because R19mab has high sensitivity, it can conveniently and accurately count low abundance CD19 CAR positive cells, which is very important for detecting CD19 CAR-T cells *in vivo,* monitoring clinical treatment and auxiliary diagnosis.

### (3) Immunofluorescence detection

CD19 CAR-T cell were washed with 1×PBS and then fixed in 4% paraformaldehyde at room temperature for 20 mins. The cells were washed with 1×PBS followed by incubation with a blocking solution (PBS containing 10% donkey serum) at room temperature for 30 minutes. Cells were washed with 1×PBS and incubated with R19mab for 1 hour. After washing with 1×PBS, incubation was performed with goat anti-rabbit IgG (H + L)-Cy3 secondary antibody for 30 minutes. After smearing, the cells were observed and photographed with confocal microscope.

### Results:

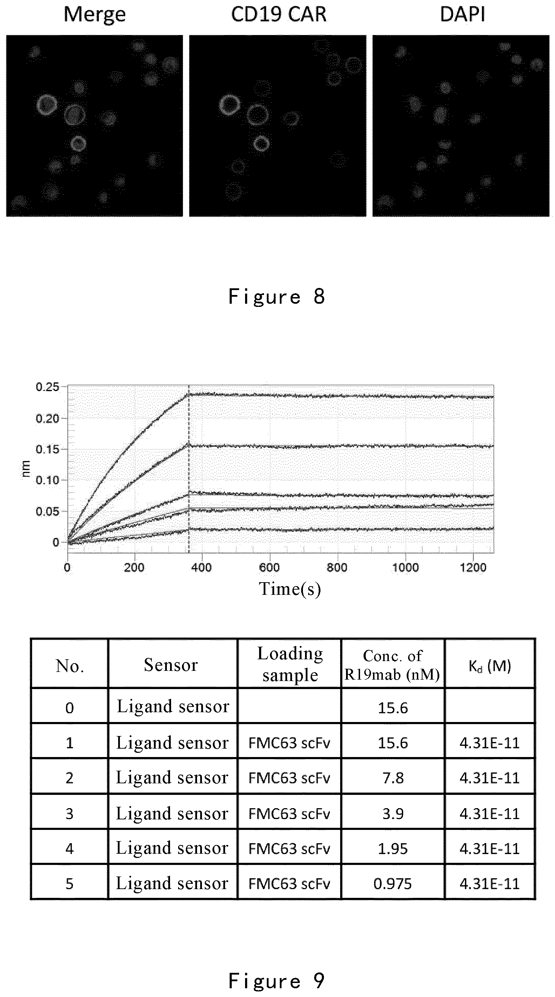

As shown in Figure 8, R19mab can clearly stain CD19 CAR positive T cells, and the red signal on the membrane represents the localization of CD19 CAR on the cell membrane. There is no red fluorescence signal on CD19 CAR negative T cells. Blue fluorescence represents nucleus.

### (4) Analysis of binding kinetics with Fortebio Octet

Fortebio Octet was used to complete the kinetic analysis of R19mab binding to FMC63 scFv. In short, the biosensor was balanced offline in PBS buffer for 600 seconds, and then detected online for 60 seconds to establish a baseline. The purified FMC63 scFv recombinant protein was diluted to 50 µg/mL with PBS and then loaded onto the sensor for 60 seconds. Sensor loaded with FMC63 scFv was then exposed to different concentrations (from 15.6 nM to 0.975 nM, 5 concentrations) of R19mab for 600 seconds, and then the sensor was transferred to PBS to dissociate for 900 seconds for dissociation rate measurement. Data Analysis 6.2 evaluation software was used to analyze the data, and a 1: 1 combination model was used for kinetic analysis.

### Results:

As shown in Figure 9, the equilibrium dissociation constant K_{D} value of R19mab binding to FMC63 scFv reaches 43 pM, indicating that R19mab binds to FMC63 scFv with a high affinity. This result is consistent with the above-mentioned R19mab sensitivity detection results.

### EXAMPLE 4. Flow cytometric detection kit for CD19 CAR positive cells using R19mab

(1) Preparing a kit (direct labeling method), which comprises the following reagents:
   (a) a first container and a fluorescent labeled R19mab located in the first container (wherein the fluorescence can be different fluorescence such as FITC, PE, Alexa Fluor647, etc.);
   (b) a second container and a NC (negative control reagent) located in the second container

The kit is used as follows:
(I) 2×10⁵ cells to be tested were taken and washed twice with PBS (containing 1% blocking agent).
(II) The cells were resuspended with 100 µL PBS (containing 1 µL fluorescent labeled R19mab), and placed at 4°C for 45 minutes.
(III) Washed twice with PBS.
(IV) Resuspended with 200µL PBS and detected by flow cytometry.

The kit is used to detect CD19 CAR-T cells, and the results show that the kit has the effects of high sensitivity and good specificity.

(2) Preparing a kit (secondary antibody method), which comprises the following agents:
(a) a first container and R19mab located in the first container;
(a) a second container and a fluorescent labeled secondary antibody located in the second container (wherein the fluorescence can be different fluorescence such as FITC, PE, Alexa Fluor647, etc.);
(c) a third container and a NC (negative control agent) located in the third container; and
(d) a fourth container and a blocking agent located in the fourth container.

The kit is used as follows:
(I) 2×10⁵ cells to be tested were taken and washed twice with PBS (containing 1% blocking agent).
(II) The cells were resuspended with 100 µL PBS (containing 1% blocking agent, 1 µL R19mab), and placed at 4°C for 45 minutes.

(3) Washed twice with PBS.

(4) The cells were resuspended with 100 µL PBS (containing 0.1 µL fluorescent secondary antibody), and placed at 4°C for 30 minutes in the dark.

(5) Washed with PBS twice.

(6) Resuspended with 200µL PBS and detected by flow cytometry.

The kit was used to detect CD19 CAR-T cells, and the results show that the kit had the effects of high sensitivity and good specificity.

**Table 1. Antibody used in the experiment**

| Antibody | Cat. No. | Brand |
|---|---|---|
| R19mab | NA | BioSwan |
| FMC63 | MAB 1794 | Merck & Millipore |
| Anti-rabbit IgG-peroxidase | A0545 | Sigma-Aldrich |
| Biotinylated Protein L. | M00097 | Genscript |
| Biotinylated CD19/Fc | CD9-H8259 | ACROBiosystems |
| Streptavidin, PE | 12-4317-87 | eBioscience |
| Streptavidin, FITC | 405201 | BioLegend |
| Goat anti-rabbit IgG (H + L), Alexa Fluor 647 | A-21245 | Invitrogen |
| Goat anti-rabbit IgG (H + L), Alexa Fluor 488 | A-11034 | Invitrogen |
| Goat anti-rabbit IgG (H + L), Cy3 | 111-165-045 | Jackson ImmunoResearch |

## Claims

1. An antibody having:
(1) a heavy chain variable region comprising the following three complementarity determining regions or CDRs:
CDR1 as shown in SEQ ID NO: 3;
CDR2 as shown in SEQ ID NO: 4; and
CDR3 as shown in SEQ ID NO: 5; and
(2) a light chain variable region comprising the following three complementarity determining regions or CDRs:
CDR1' as shown in SEQ ID NO: 6;
CDR2' as shown in SEQ ID NO: 7, and
CDR3' as shown in SEQ ID NO: 8,
wherein the antibody is an antibody specific against the CD19 antibody, and the CD19 antibody is a FMC63 scFv.

2. The antibody of claim 1, wherein the heavy chain variable region has the amino acid sequence as shown in SEQ ID NO: 9.

3. The antibody of claim 1, wherein the light chain variable region has the amino acid sequence as shown in SEQ ID NO: 10.

4. The antibody of any one of claims 1-3, wherein the antibody comprises a single chain antibody (scFv), a Fab or (Fab ')₂ fragment, a double-chain antibody, or a monoclonal antibody.

5. A recombinant protein having:
(i) the antibody of any one of claims 1-4; and
(ii) an optional tag sequence assisting expression and/or purification.

6. An immunoconjugate comprising:
(a) the antibody of any one of claims 1-4; and
(b) a coupling moiety selected from the group consisting of a detectable label, a drug, a toxin, a cytokine, a radionuclide, an enzyme, a magnetic bead, or agarose.

7. A detection reagent for detecting CD19 CAR positive cells comprising: a first antibody which is the antibody of any one of claims 1-4

8. The detection reagent of claim 7 wherein the detection reagent further comprises a second detection reagent specifically binding to the first antibody or a label coupled to the first antibody.

9. A CD19 CAR detection kit comprising a first container containing the antibody of any one of claims 1-4 and optional instructions.

10. Use of the detection reagent of claim 7 for preparing a reagent or kit for detecting CD19 CAR positive cells *in vitro*

11. An *in vitro* method for detecting CD19 CAR positive cells, comprising the steps of:
(I) providing the detection reagent of claim 7;
(II) detecting a cell population to be tested with the detection reagent, thereby obtaining a qualitative or quantitative detection result of CD19 CAR positive cells.

## Patentansprüche

1. Antikörper, aufweisend:
(1) eine variable Region der schweren Kette, die die folgenden drei komplementaritätsbestimmenden Regionen oder CDRs umfasst:
CDR1 gemäß SEQ ID NO: 3;
CDR2 gemäß SEQ ID NO: 4; und
CDR3 gemäß SEQ ID NO: 5; und
(2) eine variable Region der leichten Kette, die die folgenden drei komplementaritätsbestimmenden Regionen oder CDRs umfasst:
CDR1' gemäß SEQ ID NO: 6;
CDR2' gemäß SEQ ID NO: 7 und
CDR3' gemäß SEQ ID NO: 8,
wobei es sich bei dem Antikörper um einen gegen den CD19-Antikörper spezifischen Antikörper und bei dem CD19-Antikörper um ein FMC63-scFv handelt.

2. Antikörper nach Anspruch 1, wobei die variable Region der schweren Kette die Aminosäuresequenz gemäß SEQ ID NO: 9 aufweist.

3. Antikörper nach Anspruch 1, wobei die variable Region der leichten Kette die Aminosäuresequenz gemäß SEQ ID NO: 10 aufweist.

4. Antikörper nach einem der Ansprüche 1-3, wobei der Antikörper einen einkettigen Antikörper (scFv), ein Fab- oder (Fab ')₂-Fragment, einen doppelkettigen Antikörper oder einen monoklonalen Antikörper umfasst.

5. Rekombinantes Protein, aufweisend:
(i) den Antikörper nach einem der Ansprüche 1-4; und
(ii) eine optionale Tag-Sequenz, die die Expression und/oder Reinigung unterstützt.

6. Immunkonjugat, umfassend:
(a) den Antikörper nach einem der Ansprüche 1-4; und
(b) eine Kopplungsgruppierung, die ausgewählt ist aus der Gruppe bestehend aus einer nachweisbaren Markierung, einem Arzneistoff, einem Toxin, einem Cytokin, einem Radionuklid, einem Enzym, einem Magnetkügelchen oder Agarose.

7. Nachweisreagenz zum Nachweisen CD19-CAR-positiver Zellen, umfassend:
einen ersten Antikörper, bei dem es sich um den Antikörper nach einem der Ansprüche 1-4 handelt.

8. Nachweisreagenz nach Anspruch 7, wobei das Nachweisreagenz ferner ein zweites Nachweisreagenz umfasst, das an den ersten Antikörper oder eine an den ersten Antikörper gekoppelte Markierung spezifisch bindet.

9. CD19-CAR-Nachweiskit, umfassend einen ersten Behälter, der den Antikörper nach einem der Ansprüche 1-4 und optionale Anweisungen enthält.

10. Verwendung des Nachweisreagenz nach Anspruch 7 zur Herstellung eines Reagenz oder Kits zum Nachweis CD19-CAR-positiver Zellen in vitro.

11. In-vitro-Verfahren zum Nachweis CD19-CAR-positiver Zellen, umfassend die Schritte:
(I) Bereitstellen des Nachweisreagenz nach Anspruch 7;
(II) Nachweisen einer zu testenden Zellpopulation mit dem Nachweisreagenz, wodurch ein qualitatives oder quantitatives Nachweisergebnis von CD19-CAR-positiven Zellen erhalten wird.

## Revendications

1. Anticorps ayant :
(1) une région variable de chaîne lourde comprenant les trois régions déterminant la complémentarité ou CDR suivantes :
CDR1 comme indiquée dans SEQ ID NO: 3 ;
CDR2 comme indiquée dans SEQ ID NO: 4 ; et
CDR3, comme indiquée dans SEQ ID NO: 5 ; et
(2) une région variable de chaîne légère comprenant les trois régions déterminant la complémentarité ou CDR suivantes :
CDR1' comme indiquée dans SEQ ID NO: 6 ;
CDR2' comme indiquée dans SEQ ID NO: 7, et
CDR3' comme indiquée dans SEQ ID NO: 8,
dans lequel l'anticorps est un anticorps spécifique contre l'anticorps CD19, et l'anticorps CD19 est un scFv FMC63.

2. Anticorps selon la revendication 1, dans lequel la région variable de chaîne lourde a la séquence d'acides aminés telle que représentée dans SEQ ID NO: 9.

3. Anticorps selon la revendication 1, dans lequel la région variable de chaîne légère a la séquence d'acides aminés telle que représentée dans SEQ ID NO: 10.

4. Anticorps selon l'une quelconque des revendications 1 à 3, dans lequel l'anticorps comprend un anticorps monocaténaire (scFv), un fragment Fab ou (Fab')₂, un anticorps bicaténaire ou un anticorps monoclonal.

5. Protéine recombinante présentant :
(i) l'anticorps selon l'une quelconque des revendications 1 à 4 ; et
(ii) une séquence d'étiquette facultative aidant à l'expression et/ou la purification.

6. Immunoconjugué comprenant :
(a) l'anticorps selon l'une quelconque des revendications 1 à 4 ; et
(b) un fragment de couplage choisi dans le groupe constitué par un marqueur détectable, un médicament, une toxine, une cytokine, un radionucléide, une enzyme, une bille magnétique, ou de l'agarose.

7. Réactif de détection pour la détection de cellules positives pour CAR CD19 comprenant :
un premier anticorps qui est l'anticorps selon l'une quelconque des revendications 1 à 4.

8. Réactif de détection selon la revendication 7, dans lequel le réactif de détection comprend en outre un second réactif de détection se liant spécifiquement au premier anticorps ou un marqueur couplé au premier anticorps.

9. Kit de détection de CAR CD19 comprenant un premier récipient contenant l'anticorps selon l'une quelconque des revendications 1 à 4, et des instructions facultatives.

10. Utilisation du réactif de détection selon la revendication 7 pour préparer un réactif ou un kit pour détecter des cellules positives pour CAR CD19 *in vitro.*

11. Procédé *in vitro* de détection de cellules positives pour CAR CD19, comprenant les étapes de :
(I) fourniture du réactif de détection de la revendication 7 ;
(II) détection d'une population cellulaire à tester avec le réactif de détection, ce qui permet d'obtenir un résultat de détection qualitatif ou quantitatif de cellules positives pour CAR CD19.
